(19) Europäisches Patentamt
European Patent Office
Office européen des brevets

(11) EP 4 361 260 A1

(12) EUROPEAN PATENT APPLICATION
published in accordance with Art. 153(4) EPC

(43) Date of publication:
**01.05.2024 Bulletin 2024/18**

(21) Application number: **22827500.4**

(22) Date of filing: **20.06.2022**

(51) International Patent Classification (IPC):
***C12N 9/06*** (2006.01) ***C12N 15/53*** (2006.01)
***C12N 11/089*** (2020.01) ***C12N 1/21*** (2006.01)
***C12P 17/16*** (2006.01)

(52) Cooperative Patent Classification (CPC):
**C12N 9/0004; C12N 11/089; C12N 15/66; C12N 15/70; C12N 15/74; C12P 17/16; C12P 41/00;** C12R 2001/19

(86) International application number:
**PCT/CN2022/099711**

(87) International publication number:
**WO 2022/268006 (29.12.2022 Gazette 2022/52)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **20.06.2021 CN 202110682123**
**21.06.2021 CN 202110684461**

(71) Applicant: **Porton Pharma Solutions Ltd.**
**Chongqing 401220 (CN)**

(72) Inventor: **CHEN, Zecong**
**Chongqing 401120 (CN)**

(74) Representative: **Scholl, Matthias**
**Friedrichstraße 114a**
**10117 Berlin (DE)**

Remarks:
The complete document including Reference Table(s) and the Sequence Listing(s) can be downloaded from the EPO website

# (54) IMINE REDUCTASE MUTANT, CO-EXPRESSED ENZYME OF IMINE REDUCTASE AND GLUCOSE DEHYDROGENASE, AND APPLICATION THEREOF

(57) Disclosed in the present invention are an imine reductase mutant, and a method for preparing (S)-nicotine using the imine reductase mutant. The first objective of the invention is to provide an imine reductase mutant, for solving the problems in the prior art that no nicotine of S configuration can be obtained by using an enzyme and the enzyme activity is low, and this mutant can implement catalytic reduction of a substrate myosmine or 4-(Methylamino)-1-(pyridin-3-yl)butanone to (S)-nicotine. The second objective of the present invention is to provide a coexpressed enzyme of the imine reductase and a glucose dehydrogenase, specifically being a co-expressed enzyme of an imine reductase mutant enzyme and the glucose dehydrogenase. The co-expressed enzyme is used to prepare (S)-nicotine, and the cost of enzymatically preparing (S)-nicotine is lowered.

EP 4 361 260 A1

## Description

BACKGROUND

The disclosure relates to the field of pharmaceutical and chemical engineering, and more specifically, to an imine reductase mutant, a co-expressed recombinant enzyme of the imine reductase and glucose dehydrogenase, and applications thereof.

(S)-Nicotine is a naturally occurring alkaloid present in Solanaceae plants, notably as a substantial component in tobacco. Although chemical synthesis methods exist for producing (R,S)-nicotine, an additional separation step is required to obtain pure (S)-nicotine. The chemical synthesis methods result in higher costs compared to the extraction of nicotine from tobacco leaves.

Employing enzyme catalysis to prepare (S)-nicotine is environmentally friendly and cost-effective.

In 2010, Koichi Mitsukura et al. (Org. Biomol. Chem., 2010, 8, 4533-4535) identified two imine reductases, one from *Streptomyces sp.* GF3587 and another from *Streptomyces sp.* GF 3546. The two imine reductases were employed to catalyze the reduction of 2-methylpyridine (2-MPN) into both S-2-methylpyridine (S-2MP) and R-2-methylpyridine (R-2MP).

Patent WO2014174505 discloses a method for preparing nicotine using Streptomyces sp. GF3587 and 3546. However, the substrate, 4-(methylamino)-1-(pyridin-3-yl)butan-1-one, exhibits a conversion rate of just 23% and exclusively yields (R)-nicotine as a final product.

Patent WO2020098978 discloses a method for preparing (S)-nicotine using an imine reductase. The imine reductase catalyzes the conversion of myosmine to nornicotine. Subsequently, nornicotine is methylated to yield (S)-nicotine. The imine reductase, obtained from Enzymicals, achieves a conversion rate of up to 77% after 8 hours and 99% after 24 hours.

Hence, there is a need for a new imine reductase that can effectively convert the substrate to into (S)-nicotine, exhibiting increased enzyme activity, enhanced conversion rates, and optical purity, particularly at higher initial substrate concentrations.

The imine reductases are reliant on the coenzyme NADPH as a reducing agent to perform the reductive functions. Nevertheless, in patents WO2014174505 and WO2020098978, an imine reductase and glucose dehydrogenase are prepared independently and then combined for use, resulting in a more complex enzyme preparation process and increased enzyme costs. Li Jixuan et al. (Biotechnology Bulletin, 2019, 35(1): 105-111) developed a co-expression system for S-imine reductase and glucose dehydrogenase, reducing the enzyme expenses by allowing both enzymes to be produced simultaneously. However, the co-expression system is specifically designed for converting 2-MPN into S-2MP. Additionally, glucose dehydrogenase typically has higher activity than the imine reductase, and in the co-expression system, the imine reductase and glucose dehydrogenase are present in equal amounts, leading to an excess of residue glucose dehydrogenase. This causes emulsification issues during practical production. To address the problem, a more active imine reductase is required for co-expression with glucose dehydrogenase to facilitate the conversion of the substrate into (S)-nicotine.

SUMMARY

To solve the aforesaid problems, the first objective of the disclosure is to provide an imine reductase mutant that catalytically reduces a substrate, myosmine or 4-(methylamino)-1-(pyridin-3-yl)butan-1-one, to (S)-nicotine.

The second objective of the disclosure is to provide a co-expressed recombinant enzyme that simultaneously expresses the imine reductase mutant and glucose dehydrogenase. The co-expressed recombinant enzyme is used to produce (S)-nicotine, thereby reducing the expenses associated with enzymatic preparation of (S)-nicotine.

The technical solution of the disclosure is as follows:

The imine reductase mutant comprises a mutation in amino acid residue compared to an amino acid sequence represented by **SEQ ID NO:** 1; and the mutation comprises V171, A172, Y230, or a combination thereof.

The amino acid sequence represented by **SEQ ID NO: 1** is derived from a wild-type imine reductase originating from Aeromonas veronii. The imine reductase mutant is obtained through alternation of the wild-type imine reductase via the mutation comprising V171, A172, Y230, or a combination thereof. The imine reductase mutant exhibits an increased activity of 31 to 50-fold when catalyzing the substrate, myosmine, as compared to the wild-type imine reductase.

In a class of this embodiment, the mutation in the amino acid sequence comprises V171Y/N/A/S, A172V/F, Y230G/A/T, or a combination thereof.

The imine reductase mutant comprises one of amino acid sequences selecting from a group consisting of **SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO:**

**45, SEQ ID NO: 47, SEQ ID NO: 49, SEQ ID NO: 51, SEQ ID NO: 53, SEQ ID NO: 55, SEQ ID NO: 57, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 87, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 105, SEQ ID NO: 107, SEQ ID NO: 109, SEQ ID NO: 111, SEQ ID NO: 113, SEQ ID NO: 115, SEQ ID NO: 117, SEQ ID NO: 119, SEQ ID NO: 121, SEQ ID NO: 123, SEQ ID NO: 125, SEQ ID NO: 127, SEQ ID NO: 129, SEQ ID NO: 131, SEQ ID NO: 133, SEQ ID NO: 135, SEQ ID NO: 137, SEQ ID NO: 139, SEQ ID NO: 141, SEQ ID NO: 143, SEQ ID NO: 145, SEQ ID NO: 147, SEQ ID NO: 149, SEQ ID NO: 151, SEQ ID NO: 153, SEQ ID NO: 155, SEQ ID NO: 157, SEQ ID NO: 159, SEQ ID NO: 161, SEQ ID NO: 163, SEQ ID NO: 165,** and **SEQ ID NO: 167.**

The disclosure further provides a method for preparing (S)-nicotine, and the method comprises; under suitable conditions, catalytically reducing a substrate I by the imine reductase mutant, and methylating (S)-nornicotine; and the substrate I is shown in formula I:

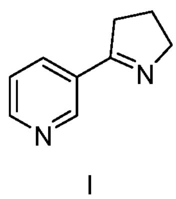

I .

Under the suitable conditions, the substrate I, coenzyme, glucose, glucose dehydrogenase, a buffer solution, and the imine reductase mutant are combined to form a mixture, and (S)-nornicotine is obtained as a reaction product; the substrate I is myosmine; and (S)-nornicotine is shown in formula III:

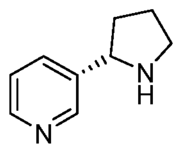

III

Preferably, the substrate I is loaded at a concentration of 10-300 g/L; a concentration of the imine reductase mutant is between 1-10 g/L; a concentration of the coenzyme is 1 g/L; a concentration of glucose dehydrogenase is 2 g/L; a pH of the mixture is maintained within a range of 6 to 7; the reaction is conducted at a temperature of 23°C - 30°C; and the reaction is conducted for 15-24 hours.

Preferably, the substrate I is loaded at a concentration of 10 g/L, 50 g/L, 100 g/L, 150 g/L, 200 g/L, 250 g/L, or 300 g/L.

Preferably, the concentration of the imine reductase mutant is 1 g/L, 2 g/L, 3 g/L, 4 g/L, 5 g/L, 6 g/L, 7 g/L, 8 g/L, 9 g/L, or 10 g/L.

Preferably, the reaction is conducted at a temperature of 23°C, 24°C, 25°C, 26°C, 27°C, 28°C, 29°C, or 30°C.

Preferably, the reaction is conducted for 15 hours, 16 hours, 17 hours, 18 hours, 19 hours, 20 hours, 21 hours, 22 hours, 23 hours, or 24 hours.

(S)-nornicotine is then methylated to yield (S)-nicotine. For instance, (S)-nornicotine reacts with formic acid/formaldehyde to produce (S)-nicotine.

The method for preparing (S)-nicotine is described as follows:

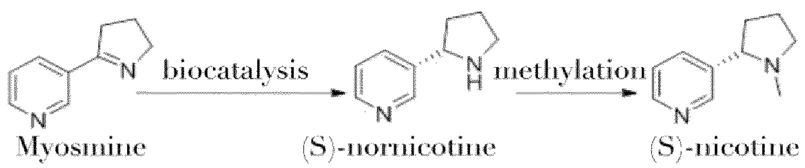


The disclosure further provides another method for preparing (S)-nicotine, and the method comprises: ring-closing and then catalytically reducing a substrate II by the imine reductase mutant, or;

desalting, ring-closing, and catalytically reducing a salt of the substrate II by the imine reductase mutant;

and the substrate II is shown in formula II:

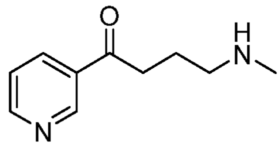

II

.

The salt of the substrate II comprises hydrochloride, dihydrochloride, hydrobromide, dihydrobromide, sulphate or hydrogen sulphate.

After the ring-closure, the substrate II is transformed into an enamine compound or an iminium cation compound, represented by the following chemical formulas:

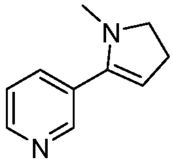

or

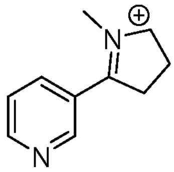

Under suitable conditions, the substrate II, coenzyme, glucose, glucose dehydrogenase, a buffer solution, and the imine reductase mutant are combined to form a mixture, and (S)-nicotine is obtained as a reaction product.

Preferably, the substrate II is loaded at a concentration of 10-300 g/L; a concentration of the imine reductase mutant is between 1-10 g/L; a concentration of the coenzyme is 1 g/L; a concentration of glucose dehydrogenase is 2 g/L; a pH of the mixture is maintained within a range of 6 to 7; the reaction is conducted at a temperature of 23°C to 30°C; and the reaction is conducted for 15-24 hours.

Preferably, the substrate II is loaded at a concentration of 10 g/L, 50 g/L, 100 g/L, 150 g/L, 200 g/L, 250 g/L, or 300 g/L.

Preferably, the concentration of the imine reductase mutant is 1 g/L, 2 g/L, 3 g/L, 4 g/L, 5 g/L, 6 g/L, 7 g/L, 8 g/L, 9 g/L, or 10 g/L.

Preferably, the reaction is conducted at a temperature of 23°C, 24°C, 25°C, 26°C, 27°C, 28°C, 29°C, or 30°C.

Preferably, the reaction is conducted for 15 hours, 16 hours, 17 hours, 18 hours, 19 hours, 20 hours, 21 hours, 22 hours, 23 hours, or 24 hours.

The imine reductase mutant comprises one of amino acid sequences selecting from a group consisting of **SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 55, SEQ ID NO: 57, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 87, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 105, SEQ ID NO: 107, SEQ ID NO: 109, SEQ ID NO: 111, SEQ ID NO: 113, SEQ ID NO: 115, SEQ ID NO: 117, SEQ ID NO: 119, SEQ ID NO: 121, SEQ ID NO: 123, SEQ ID NO: 125, SEQ ID NO: 127, SEQ ID NO: 129, SEQ ID NO: 131, SEQ ID NO: 133, SEQ ID NO: 135, SEQ ID NO: 137, SEQ ID NO: 139, SEQ ID NO: 141, SEQ ID NO: 143, SEQ ID NO: 145, SEQ ID NO: 147, SEQ ID NO: 149, SEQ ID NO: 151, SEQ ID NO: 153, SEQ ID NO: 155, SEQ ID NO: 157, SEQ ID NO: 159, SEQ ID NO: 161, SEQ ID NO: 163, SEQ ID NO: 165, and SEQ ID NO: 167.**

The nucleic acid sequences corresponding to the amino acid sequences are as follows: **SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, SEQ ID NO: 56, SEQ ID NO: 58, SEQ ID NO: 60, SEQ ID NO: 62, SEQ ID NO: 64, SEQ ID NO: 66, SEQ ID NO: 68, SEQ ID NO: 70, SEQ ID NO: 72, SEQ ID NO: 74, SEQ ID NO: 76, SEQ ID NO: 78, SEQ ID NO: 80, SEQ ID NO: 82, SEQ ID NO: 84, SEQ ID NO: 86, SEQ ID NO: 88, SEQ ID NO: 90, SEQ ID NO: 92, SEQ ID NO: 94, SEQ ID NO: 96, SEQ ID NO: 98, SEQ ID NO: 100, SEQ ID NO: 102, SEQ**

**ID NO: 104, SEQ ID NO: 106, SEQ ID NO: 108, SEQ ID NO: 110, SEQ ID NO: 112, SEQ ID NO: 114, SEQ ID NO: 116, SEQ ID NO: 118, SEQ ID NO: 120, SEQ ID NO: 122, SEQ ID NO: 124, SEQ ID NO: 126, SEQ ID NO: 128, SEQ ID NO: 130, SEQ ID NO: 132, SEQ ID NO: 134, SEQ ID NO: 136, SEQ ID NO: 138, SEQ ID NO: 140, SEQ ID NO: 142, SEQ ID NO: 144, SEQ ID NO: 146, SEQ ID NO: 148, SEQ ID NO: 150, SEQ ID NO: 152, SEQ ID NO: 154, SEQ ID NO: 156, SEQ ID NO: 158, SEQ ID NO: 160, SEQ ID NO: 162, SEQ ID NO: 164, SEQ ID NO: 166,** and **SEQ ID NO: 168.**

The disclosure further provides an expression vector; the expression vector comprises an imine reductase mutant gene fragment comprising one of the nucleic acid sequences.

The disclosure further provides a vector cell comprising the expression vector.

The disclosure further provides a method for preparing the imine reductase mutant.

The imine reductase mutant and glucose dehydrogenase are simultaneously expressed through a co-expressed recombinant enzyme.

The co-expressed recombinant enzyme is generated by a cell comprising both the imine reductase mutant gene fragment and a glucose dehydrogenase gene fragment.

Specifically, the imine reductase mutant gene fragment and the glucose dehydrogenase are inserted into a plasmid, such as pET-30a(+), to prepare a recombinant vector.

The recombinant vector is transferred into engineered bacteria, such as Escherichia coli BL21(DE3), to obtain a vector cell.

The imine reductase mutant gene fragment and the glucose dehydrogenase gene fragment are expressed in the same engineered bacteria to prepare the co-expressed recombinant enzyme. The co-expressed recombinant enzyme effectively addresses the cost-related challenges associated with separately preparing glucose dehydrogenase, while allowing for the repeated utilization of coenzymes in a co-expression system.

A method for preparing the co-expressed recombinant enzyme, and the method comprises: amplifying the glucose dehydrogenase gene fragment from a vector using a pair of primers comprising specific restriction enzyme recognition sites; digesting the imine reductase mutant gene fragment on the expression vector and the amplified glucose dehydrogenase gene fragment with two restriction enzymes, BamHI and XhoI; recovering the two digested gene fragments from an agarose gel; ligating the two digested gene fragments using T4 DNA ligase to form a ligated DNA; transforming the ligated DNA into Escherichia coli BL21 competent cells, thus obtaining recombinant bacteria containing both the imine reductase mutant gene fragment and the glucose dehydrogenase gene fragment; cultivating, inducing, and centrifuging the recombinant bacteria and collecting bacterial cells; resuspending and ultrasonically breaking the bacterial cells; and freeze-drying a resulting solution to obtain powders of the co-expressed recombinant enzyme.

A method for preparing(S)-nicotine by the co-expressed recombinant enzyme, and the method comprises: under suitable conditions, catalytically reducing the substrate I or the substrate II by the co-expressed recombinant enzyme.

In a class of this embodiment, the substrate I, coenzyme, glucose, buffer solution, and the co-expressed recombinant enzyme are combined to form a mixture; (S)-nornicotine is obtained as a reaction product and then methylated to yield (S)-nicotine.

In a class of this embodiment, the substrate II is cyclized and then catalyzed by the co-expressed recombinant enzyme to yield (S)-nicotine, or;

a salt of the substrate II is desalted, cyclized, and catalyzed by the coexpressed recombinant enzyme to yield (S)-nicotine.

The salt of the substrate II comprises hydrochloride, dihydrochloride, hydrobromide, dihydrobromide, sulphate or hydrogen sulphate.

The following advantages are associated with the disclosure:

1. The imine reductase mutant catalyzes the substrate II or the salt thereof into (S)-nicotine with a conversion rate of 99.9% and a chemical purity of 99.9%; and the substrate is loaded at a concentration of 300 g/L.

2. The imine reductase mutant catalyzes the substrate I into (S)-nicotine, demonstrating enhanced enzymatic activity while concurrently increasing the substrate load.

3. Through the utilization of the co-expressed recombinant enzyme, both the imine reductase mutant and glucose dehydrogenase are co-expressed in the same engineered bacterium, thereby producing (S)-nicotine. The co-expression system enables coenzyme recycling, eliminating the necessity for a separate step in glucose dehydrogenase preparation, thus resulting in cost reduction.

BRIEF DESCRIPTION OF THE DRAWINGS

FIG. **1** is a synthesis route of (S)-(3-pyrrolidin-2-yl)pyridine;

FIG. **2** is a reverse-phase, non-chiral HPLC chromatogram of (S)-(3-pyrrolidin-2-yl)pyridine standard;

FIG. **3** is a reverse-phase, non-chiral HPLC chromatogram of nicotine standard as a substrate;

FIG. **4** is a reverse-phase, non-chiral HPLC chromatogram of the reaction control in Example **5** (in which the imine reductase mutant comprises an amino acid sequence represented by **SEQ ID NO: 1**);

FIG. **5** is a normal-phase, chiral HPLC chromatogram of (R,S)-(3-pyrrolidin-2-yl)pyridine;

FIG. **6** is a normal-phase, chiral HPLC chromatogram of (S)-(3-pyrrolidin-2-yl)pyridine standard;

FIG. **7** is a normal-phase, chiral HPLC chromatogram of (S)-(3-pyrrolidin-2-yl)pyridine synthesized in Example **5** (in which the imine reductase mutant comprises an amino acid sequence represented by **SEQ ID NO: 1**) of the disclosure;

FIG. **8** is a normal-phase, chiral HPLC chromatogram of (S)-(3-pyrrolidin-2-yl)pyridine synthesized in Example **5** (in which the imine reductase mutant comprises an amino acid sequence represented by **SEQ ID NO: 17**) of the disclosure; and

FIG. **9** is a plasmid map of a co-expressed recombinant bacteria according to one example of the disclosure.

DETAILED DESCRIPTION

To further illustrate the disclosure, embodiments detailing the imine reductase mutant, the co-expressed recombinant enzyme, and applications thereof are described below. It should be noted that the following embodiments are intended to describe and not to limit the disclosure.

When specific techniques or conditions are not explicitly stated in the examples, standard practices from the relevant literature or product specifications shall be used. Generic reagents and instruments, not specifying the manufacturer, can be acquired from trusted suppliers.

**Example 1**

Expression of imine reductase and preparation of a powder thereof

(1) A gene, encoding wild-type imine reductase from Aeromonas veronii, represented by a nucleic acid sequence shown in **SEQ ID NO: 2,** was optimized for codon usage and fully synthesized by Nanjing GenScript Corporation. The optimized gene was then inserted into the pET-30a(+) plasmid. The resulting recombinant plasmid was introduced into Escherichia coli BL21(DE3), plated on an LB agar plate containing 50 μg/mL kanamycin, and incubated overnight at 37°C. Specific bacterial colonies were selected and confirmed through DNA sequencing. The confirmed transformants were denoted as recombinant E. coli BL21/pET30a-No.2.

(2) The recombinant E. coli BL21/pET30a-No.2 were inoculated into 5 mL of LB liquid medium containing 50 μg/mL kanamycin and cultured overnight at 37°C. Next, 1 mL of the bacterial culture was transferred into 100 mL of LB liquid medium containing 50 μg/mL kanamycin and cultured at 37°C for 3 hours. Then, 50 μL of 1 M Isopropyl β-D-1-thiogalactopyranoside (IPTG) solution was added, and the bacterial culture was further incubated at 25°C for 16 hours. The bacterial cells were centrifuged (at 4000 rpm, 4°C, 10 min) to yield a pellet. The pellet was resuspended in PBS buffer (with a pH value of 7.0), using a volume four times that of the pellet. The suspension was ultrasonically treated, and the resulting solution was freeze-dried to yield a powder of imine reductase. The amino acid sequence corresponding to the wild-type imine reductase is represented by **SEQ ID NO: 1.**

**Example 2**

Construction of a mutant library of imine reductase

A gene, encoding wild-type imine reductase, represented by an amino acid sequence shown in **SEQ ID NO:**

**1,** was used as a template. Error-prone PCR was conducted using a random mutagenesis kit (Beijing Biomed) and a pair of primers listed Table **1.** The resulting PCR products were then recovered using a DNA fragment recovery kit (Shanghai BioTech) in accordance with the manufacturer's instructions.

The recovered PCR product and the pET-30a(+) plasmid were separately digested with two restriction enzymes, NdeI and BamHI. The digested products were recovered and ligated using T4 DNA ligase into linearized pET-30a(+) plasmids at 22°C for 1 hour. The ligation products were introduced into Escherichia coli BL21 (DE3) competent cells (Shanghai BioTech) and cultured overnight at 37°C, thereby generating a random mutant library.

Table **1** Primer sequences for error-prone PCR

| Sequence number | Primer sequence |
|---|---|
| **SEQ ID NO: 169** | TATACATATGCGCCATCTGAGCGTGATTGG |
| **SEQ ID NO: 170** | TTCGGATCCTTACTGCGCCGCGCCGTTGC |

From the random mutant library, a valuable mutant comprising a nucleic acid sequence represented by **SEQ ID NO: 37** was selected as a template. The selected mutant was then subjected to site-directed saturation mutagenesis and error-prone PCR. The mutated gene was used to generate multiple rounds of the mutant library, and each round was screened to identify mutants with desirable properties or traits.

**Example 3**

Primary screening of imine reductase mutants

(1) Colonies from the random mutant were selected and placed into a 96-well microcultivation plate. Each well in the microcultivation plate contains 150 μL of LB liquid medium and 50 μg/mL kanamycin. The microcultivation plate was incubated at 37°C with continuous shaking at 220 rpm overnight. 20 μL of the bacterial culture was transferred to another 96-well plate containing 380 μL of LB liquid medium and 50 μg/mL kanamycin. The 96-well plate was then incubated at 37°C with continuous shaking at 220 rpm for 2-3 hours. The culture was induced with IPTG to reach a final concentration of 0.4 mM and was allowed to cool to 25°C for overnight cultivation (at least 16 hours).

(2) The bacterial cells from the 96-well plate were collected by centrifugation (at 10 minutes and 4000 rpm for 25°C) and then resuspended in 200 μL of a lysis solution (containing 1 g/L lysozyme and 0.5 g/L streptomycin sulfate B). The suspension was shaken at 25°C and 600 rpm for 2 hours to lyse the bacterial cells. The cell fragments were separated by centrifugation (at 4000 rpm and 4°C for 10 minutes), thereby producing a supernatant. The supernatant was a crude enzyme solution used for initial assessment of the enzyme activity.

(3) Preparation of reaction mixture: 1 mL of a myosmine solution (pH 6.0, 50 mM), 0.25 mL of an NADPH solution (20 mM), and 13.75 mL of a phosphate buffer (pH 6.0, 0.1 M) was mixed in a sample slot to obtain a reaction mixture. In each well of an enzyme plate, 150 μL of the reaction mixture was dispensed. Then, 50 μL of the crude enzyme solution was added, and an immediate change in absorbance at 340 nm was measured at 25°C.

Enzyme activity is the measure of enzyme needed to produce 1 μmol of product in one unit of time.

Enzyme solution activity, expressed as units per milliliter (U/mL), is calculated using the following formula:

$$\text{Enzyme solution activity} = \frac{\Delta A_{340} \times V_0 \times N}{6.22 \times T \times V1}$$

where, ΔA340 is the change in absorbance value; V0 is the total reaction volume; N is the dilution factor applied to the enzyme solution; T is the reaction time; V1 is the quantity of enzyme added; and 6.22 is a constant factor for the NADPH slope.

**Example 4**

Re-screening of imine reductase mutants

(1) Mutants exhibiting higher enzyme activity compared to the parent strain in Example 3 were inoculated into 100 mL of LB liquid medium containing 50 μg/mL kanamycin. The cultures were grown at 37°C with agitation until the optical density at 600 nm (OD600) reached 0.6. IPTG was then added to achieve a final concentration of 0.5 mM for induction. The induction process was performed at 25°C for 16 hours. The bacterial cells were collected by centrifugation (at 4000 rpm and 25°C for 10 minutes), and broken using an ultrasonic cell breaker (JY92-2D, Ningbo New Century Biotech Co., Ltd.). The resulting solution was separated into supernatant and pellet by centrifugation (at 10000 rpm and 4°C for 20 minutes). A portion of the supernatant was used for activity testing, while the remaining supernatant was freeze-dried to produce a powder of the imine reductase mutant.

(2) In a 5 mL centrifuge tube, 50 mg of myosmine was added as a substrate, and 2 mL of phosphate buffer (pH 6.0, 0.1 M) was mixed in. Subsequently, 0.5 mg of NADP and 90 mg of glucose were added and stirred until complete dissolution was achieved. Then, 5 mg of glucose dehydrogenase and 0.2 mL of the crude enzyme solution to be screened were added. The resulting mixture was thoroughly blended and raised to a temperature of 25°C. The reaction was stirred continuously at 300 rpm for 24 hours. After the reaction was completed, 100 μL of the reaction solution was taken and vigorously mixed with 900 μL of acetonitrile. The mixture was then filtered using a 0.22 μm filter membrane for High-Performance Liquid Chromatography (HPLC) analysis.

The mutants exhibiting higher catalytic activity compared to the parental strain were selected for sequencing. The mutations were analyzed to acquire the coding genes for the imine reductase mutants. The coding genes comprise the nucleic acid sequences represented by **SEQ ID NO: 4, SEQ ID NO: 6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, SEQ ID NO: 56, SEQ ID NO: 58, SEQ ID NO: 60, SEQ ID NO: 62, SEQ ID NO: 64, SEQ ID NO: 66, SEQ ID NO: 68, SEQ ID NO: 70, SEQ ID NO: 72, SEQ ID NO: 74, SEQ ID NO: 76, SEQ ID NO: 78, SEQ ID NO: 80, SEQ ID NO: 82, SEQ ID NO: 84, SEQ ID NO: 86, SEQ ID NO: 88, SEQ ID NO: 90, SEQ ID NO: 92, SEQ ID NO: 94, SEQ ID NO: 96, SEQ ID NO: 98, SEQ ID NO: 100, SEQ ID NO: 102, SEQ ID NO: 104, SEQ ID NO: 106, and SEQ ID NO: 108.**

The amino acid sequences corresponding to the nucleic acid sequences are as follows: **SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 55, SEQ ID NO: 57, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 87, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 105, SEQ ID NO: 107, SEQ ID NO: 109, SEQ ID NO: 111, SEQ ID NO: 113, SEQ ID NO: 115, SEQ ID NO: 117, SEQ ID NO: 119, SEQ ID NO: 121, SEQ ID NO: 123, SEQ ID NO: 125, SEQ ID NO: 127, SEQ ID NO: 129, SEQ ID NO: 131, SEQ ID NO: 133, SEQ ID NO: 135, SEQ ID NO: 137, SEQ ID NO: 139, SEQ ID NO: 141, SEQ ID NO: 143, SEQ ID NO: 145, SEQ ID NO: 147, SEQ ID NO: 149, SEQ ID NO: 151, SEQ ID NO: 153, SEQ ID NO: 155, SEQ ID NO: 157, SEQ ID NO: 159, SEQ ID NO: 161, SEQ ID NO: 163, SEQ ID NO: 165, and SEQ ID NO: 167.**

The results of activity testing for different imine reductase mutants are shown in Table **2.**

Table **2** Mutation sites and activity testing of imine reductase mutants

| Number | Mutation in amino acid residue compared to **SEQ ID NO: 1** | Activity |
|---|---|---|
| **SEQ ID NO: 1** | - | + |
| **SEQ ID NO: 3** | Y230A | ++ |
| **SEQ ID NO: 5** | V171Y | ++ |
| **SEQ ID NO: 7** | Y230T | ++ |
| **SEQ ID NO: 9** | Y230G | ++ |

(continued)

| Number | Mutation in amino acid residue compared to **SEQ ID NO: 1** | Activity |
|---|---|---|
| **SEQ ID NO: 11** | V171N | ++ |
| **SEQ ID NO: 13** | A172F | ++ |
| **SEQ ID NO: 15** | L118V, S130C, I209F, K234W | ++ |
| **SEQ ID NO: 17** | S14V, V171N | ++ |
| **SEQ ID NO: 19** | S14V, I209T | ++ |
| **SEQ ID NO: 21** | S33R, V171N, V277L | +++ |
| **SEQ ID NO: 23** | V171S | +++ |
| **SEQ ID NO: 25** | V171A | +++ |
| **SEQ ID NO: 27** | A172V | +++ |
| **SEQ ID NO: 29** | V171A, L206Y, Y230G, T235L | +++ |
| **SEQ ID NO: 31** | V171A, A172V | +++ |
| **SEQ ID NO: 33** | V171A, Y230G | +++ |
| **SEQ ID NO: 35** | A172V, Y230G | +++ |
| **SEQ ID NO: 37** | V171A, A172V, Y230G | ++++ |
| **SEQ ID NO: 39** | S14L, S33G, A172F, Q202L | ++++ |
| **SEQ ID NO: 41** | S14Q, V171A, A172V, Y230G | ++++ |
| **SEQ ID NO: 43** | S14V, S33D, C119T, V171A, A172F, Y230A, A239Y | ++++ |
| **SEQ ID NO: 45** | S14V, C119T, V171A, A172F, Y230G, T235L | ++++ |
| **SEQ ID NO: 47** | S33G, S130T, V171A, A172V, Y230G | ++++ |
| **SEQ ID NO: 49** | S14Q, L118V, V171A, A172V, Q202L, Y230A | ++++ |
| **SEQ ID NO: 51** | S14Q, S33L, L118V, V171A, A172V, Q202L, Y230T | ++++ |
| **SEQ ID NO: 53** | S14Q, S33G, C119D, S130T, V171A, A172F, Q202A, Y230G | ++++ |
| **SEQ ID NO: 55** | S14Q, S33A, S130C, V171A, A172V, L206F, Y230G, A239L | +++++ |
| **SEQ ID NO: 57** | S14Q, S33A, S130T, L118V, V171A, A172V, Y230G | +++++ |
| **SEQ ID NO: 59** | S14V, S33G, S130T, V171A, A172V, I209T, Y230G | +++++ |
| **SEQ ID NO: 61** | S33G, L118V, S130T, V171A, A172V, Y230G, K234L | +++++ |

(continued)

| Number | Mutation in amino acid residue compared to **SEQ ID NO: 1** | Activity |
|---|---|---|
| **SEQ ID NO: 63** | S33G, C119D, S130T, V171A, A172V, Q202A, Y230G, A239Y | +++++ |
| **SEQ ID NO: 65** | S14Q, S33D, S130T, V171A, A172V, Y230G, K234L | +++++ |
| **SEQ ID NO: 67** | S14V, S33G, C119T, S130T, V171A, A172F, Y230G, T235L | +++++ |
| **SEQ ID NO: 69** | S14V, S33D, C119T, S130C, V171A, A172F, Y230G, A239Y | +++++ |
| **SEQ ID NO: 71** | S14Q, S33Q, L118V, S130T, V171A, A172V, L206Y, I209T, Y230G | +++++ |
| **SEQ ID NO: 73** | S14Q, S33G, L118V, S130C, V171A, A172V, Q202L, Y230T | +++++ |
| **SEQ ID NO: 75** | S33G, C119D, S130T, V171A, A172V, Q202A, Y230G, A239L | ++++++ |
| **SEQ ID NO: 77** | S14Q, S33A, S130C, V171A, A172V, Q202S, L206F, Y230T, A239L | ++++++ |
| **SEQ ID NO: 79** | S14Q, S33G, C119D, S130T, V171A, A172V, Y230G, K234F | ++++++ |
| **SEQ ID NO: 81** | S14V, S33A, S130C, V171A, A172V, I209T, Y230G, V277L | ++++++ |
| **SEQ ID NO: 83** | S33G, L118V, S130T, V171A, A172V, Q202A, Y230G | ++++++ |
| **SEQ ID NO: 85** | S33G, L118V, S130C, V171A, A172V, Q202A, Y230G, T235S | ++++++ |
| **SEQ ID NO: 87** | S14Q, S33D, L118V, S130T, V171A, A172V, Y230G, K234L | ++++++ |
| **SEQ ID NO: 89** | S14V, S33G, C119D, S130T, V171A, A172V, Y230G, T235L, A239L | ++++++ |
| **SEQ ID NO: 91** | S14V, S33R, L118M, C119T, S130T, V171A, A172F, Y230G, A239Y | ++++++ |
| **SEQ ID NO: 93** | S14Q, S33N, L118V, S130T, V171A, A172V, L206Y, I209T, Y230G, K234L | ++++++ |
| **SEQ ID NO: 95** | S14Q, S33G, L118V, S130T, V171A, A172V, Q202L, Y230G, K234V | ++++++ |

(continued)

| Number | Mutation in amino acid residue compared to **SEQ ID NO: 1** | Activity |
|---|---|---|
| **SEQ ID NO: 97** | S14L, S33G, C119D, S130C, V171A, A172V, Q202A, I209T, Y230G, V277L | ++++++ |
| **SEQ ID NO: 99** | S14Q, S33G, S130C, V171A, A172V, L206F, Y230T, K234W, A239L | ++++++ |
| **SEQ ID NO: 101** | S14Q, S33Q, C119D, S130T, V171A, A172V, Q202Y, L206Y, Y230G, K234F, A239L | ++++++ |
| **SEQ ID NO: 103** | S14V, S33G, L118V, S130T, V171A, A172V, I209T, Y230G, K234V, T235L, A239L | ++++++ |
| **SEQ ID NO: 105** | S14Q, S33G, L118V, C119T, S130C, V171A, A172V, Q202A, I209F, Y230G, T235L, A239L | ++++++ |
| **SEQ ID NO: 107** | S14Q, S33G, L118V, S130T, V171A, A172V, Q202A, L206F, Y230G, T235L | ++++++ |
| **SEQ ID NO: 109** | V171A, A172N, Q202R | ++++ |
| **SEQ ID NO: 111** | V171A, A172F, Q202L, Y230G | ++++ |
| **SEQ ID NO: 113** | V171A, A172V, Q202L, Y230G | ++++ |
| **SEQ ID NO: 115** | S14Q, S33A, S130C, V171A, A172V, Q202L, L206F, Y230G, A239L | +++++ |
| **SEQ ID NO: 117** | S14Q, S33A, S130T, L118V, V171A, A172V, Q202L, Y230G | +++++ |
| **SEQ ID NO: 119** | S14V, S33G, S130T, V171A, A172V, Q202L, I209T, Y230G | +++++ |
| **SEQ ID NO: 121** | S33G, L118V, S130T, V171A, A172V, Q202L, Y230G, K234L | +++++ |
| **SEQ ID NO: 123** | S14Q, S33D, S130T, V171A, A172V, Q202R, Y230G, K234L | +++++ |
| **SEQ ID NO: 125** | S14V, S33G, C119T, S130T, V171A, A172N, Q202R, Y230G, T235L | +++++ |
| **SEQ ID NO: 127** | S14V, S33D, C119T, S130C, V171A, A172F, Q202R, Y230G, A239Y | +++++ |
| **SEQ ID NO: 129** | S14Q, S33Q, L118V, S130T, V171A, A172N, Q202R, L206Y, I209T, Y230G | +++++ |

(continued)

| Number | Mutation in amino acid residue compared to **SEQ ID NO: 1** | Activity |
|---|---|---|
| **SEQ ID NO: 131** | S14Q, S33G, L118V, S130C, V171A, A172V, Q202L, Y230T | +++++ |
| **SEQ ID NO: 133** | V171A, A172N, Q202R, D213E | ++++++ |
| **SEQ ID NO: 135** | S33G, C119D, S130T, V171A, A172V, Q202A, D213E, Y230G, A239L | ++++++ |
| **SEQ ID NO: 137** | S14Q, S33A, S130C, V171A, A172V, Q202S, L206F, D213E, Y230T, A239L | ++++++ |
| **SEQ ID NO: 139** | S14Q, S33G, C119D, S130T, V171A, A172V, D213E, Y230G, K234F | ++++++ |
| **SEQ ID NO: 141** | S14V, S33A, S130C, V171A, A172V, I209T, D213E, Y230G, V277L | ++++++ |
| **SEQ ID NO: 143** | S33G, L118V, S130T, V171A, A172V, Q202A, D213E, Y230G | ++++++ |
| **SEQ ID NO: 145** | S33G, L118V, S130C, V171A, A172V, Q202R, D213E, Y230G, T235S | ++++++ |
| **SEQ ID NO: 147** | S14Q, S33D, L118V, S130T, V171A, A172V, Q202R, D213E, Y230G, K234L | ++++++ |
| **SEQ ID NO: 149** | S14V, S33G, C119D, S130T, V171A, A172V, Q202R, D213C, Y230G, T235L, A239L | ++++++ |
| **SEQ ID NO: 151** | S14V, S33R, L118M, C119T, S130T, V171A, A172F, Q202R, D213C, Y230G, A239Y | ++++++ |
| **SEQ ID NO: 153** | S14Q, S33N, L118V, S130T, V171A, A172V, L206Y, I209T, D213C, Y230G, K234L | ++++++ |
| **SEQ ID NO: 155** | S14Q, S33G, L118V, S130T, V171A, A172V, Q202L, D213C, Y230G, K234V | ++++++ |
| **SEQ ID NO: 157** | S14L, S33G, C119D, S130C, V171A, A172V, Q202A, I209T, D213C, Y230G, V277L | ++++++ |
| **SEQ ID NO: 159** | S14Q, S33G, S130C, V171A, A172V, L206F, D213H, Y230T, K234W, A239L | ++++++ |

(continued)

| Number | Mutation in amino acid residue compared to **SEQ ID NO: 1** | Activity |
|---|---|---|
| **SEQ ID NO: 161** | S14Q, S33Q, C119D, S130T, V171A, A172V, Q202Y, L206Y, D213H, Y230G, K234F, A239L | ++++++ |
| **SEQ ID NO: 163** | S14V, S33G, L118V, S130T, V171A, A172V, I209T, D213H, Y230G, K234V, T235L, A239L | ++++++ |
| **SEQ ID NO: 165** | S14Q, S33G, L118V, C119T, S130C, V171A, A172V, Q202A, I209F, D213H, Y230G, T235L, A239L | ++++++ |
| **SEQ ID NO: 167** | S14Q, S33G, L118V, S130T, V171A, A172V, Q202A, L206F, D213H, Y230G, T235L | ++++++ |
| Note: '++' represents enzyme activity ranging from 1.2 to 3 times that of **SEQ ID NO: 1;** '+++° represents enzyme activity ranging from 5 to 10 times that of **SEQ ID NO: 1;** '++++' represents enzyme activity ranging from 11 to 20 times that of **SEQ ID NO: 1;** '+++++' represents enzyme activity ranging from 21 to 30 times that of **SEQ ID NO:** 1; and '++++++' represents enzyme activity ranging from 31 to 50 times that of **SEQ ID NO: 1.** | | |

**Example 5**

Application of imine reductase mutants labeled as '++' in Table **2** in the preparation of S-(3-pyrrolidin-2-yl)pyridine

In a 10 mL reaction vial, 100 mg of myosmine was added as a substrate, and 4 mL of a phosphate buffer (pH 6.0, 0.1 M) was mixed in. Subsequently, the resulting mixture was adjusted to a pH of 6.0. Then, 2.5 mg of NADP and 180 mg of glucose were added and stirred until complete dissolution was achieved. Then, 10 mg of glucose dehydrogenase and 50 mg of the powder of the imine reductase mutant were added, mixed, raised to a temperature of 25°C, and stirred at 300 rpm for 24 hours. After the reaction was completed, 100 μL of the reaction solution was taken and vigorously mixed with 900 μL of acetonitrile. The mixture was then filtered using a 0.22 μm filter membrane for HPLC analysis. 500 μL of the reaction solution was taken and adjusted to a pH exceeding 11, followed by extraction using 1000 μL of n-hexane. The extracted n-hexane layer was filtered through a membrane and used for chiral HPLC analysis. The conversion rate spectrum for **SEQ ID NO: 1** is depicted in FIG. **4,** the chiral detection spectrum for **SEQ ID NO: 1** is illustrated in FIG. **7,** and the chiral detection spectrum for **SEQ ID NO: 17** is presented in FIG. **8.**

The conversion rates and enantiomeric excess (ee%) for **SEQ ID NO: 1** and three mutants exhibiting the highest activity are shown in Table **3.**

Table **3**

| Enzyme | Conversion Rate | ee% |
|---|---|---|
| **SEQ ID NO: 1** | 46.0 | 95.4 |
| **SEQ ID NO: 9** | 78.9 | 99.2 |
| **SEQ ID NO: 11** | 83.1 | 99.6 |
| **SEQ ID NO: 17** | 90.3 | 99.9 |

**Example 6**

Application of imine reductase mutants labeled as '+++° in Table **2** in the preparation of S-(3-pyrrolidin-2-yl)pyridine

In a 10 mL reaction vial, 250 mg of myosmine was added as a substrate, and 4 mL of a phosphate buffer (pH

6.0, 0.1 M) was mixed in. Subsequently, the resulting mixture was adjusted to a pH of 6.0. Then, 2.5 mg of NADP and 450 mg of glucose were added and stirred until complete dissolution was achieved. Then, 10 mg of glucose dehydrogenase and 50 mg of the powder of the imine reductase mutant were added, mixed, raised to a temperature of 25°C, and stirred at 300 rpm for 24 hours. After the reaction was completed, 100 μL of the reaction solution was taken and vigorously mixed with 900 μL of acetonitrile. The mixture was then filtered using a 0.22 μm filter membrane for HPLC analysis. 500 μL of the reaction solution was taken and adjusted to a pH exceeding 11, followed by extraction using 1000 μL of n-hexane. The extracted n-hexane layer was filtered through a membrane and used for chiral HPLC analysis.

The conversion rates and enantiomeric excess (ee%) for **SEQ ID NO: 1** and three mutants exhibiting the highest activity are shown in Table **4.**

Table **4**

| Enzyme | Conversion Rate | ee% |
|---|---|---|
| **SEQ ID NO: 1** | 18.7 | 95.1 |
| **SEQ ID NO: 25** | 98.1 | 99.3 |
| **SEQ ID NO: 31** | 98.1 | 99.7 |
| **SEQ ID NO: 33** | 90.3 | 99.6 |

**Example 7**

Application of imine reductase mutants labeled as '++++' in Table **2** in the preparation of S-(3-pyrrolidin-2-yl)pyridine

In a 10 mL reaction vial, 500 mg of myosmine was added as a substrate, and 4 mL of a phosphate buffer (pH 6.0, 0.1 M) was mixed in. Subsequently, the resulting mixture was adjusted to a pH of 6.0. Then, 2.5 mg of NADP and 900 mg of glucose were added and stirred until complete dissolution was achieved. Then, 10 mg of glucose dehydrogenase and 50 mg of the powder of the imine reductase mutant were added, mixed, raised to a temperature of 25°C, and stirred at 300 rpm for 24 hours. After the reaction was completed, 100 μL of the reaction solution was taken and vigorously mixed with 900 μL of acetonitrile. The mixture was then filtered using a 0.22 μm filter membrane for HPLC analysis. 500 μL of the reaction solution was taken and adjusted to a pH exceeding 11, followed by extraction using 1000 μL of n-hexane. The extracted n-hexane layer was filtered through a membrane and used for chiral HPLC analysis.

The conversion rates and enantiomeric excess (ee%) for **SEQ ID NO: 1** and three mutants exhibiting the highest activity are shown in Table **5.**

Table **5**

| Enzyme | Conversion Rate | ee% |
|---|---|---|
| **SEQ ID NO: 1** | 8.9 | 95.6 |
| **SEQ ID NO: 37** | 99.9 | 99.6 |
| **SEQ ID NO: 47** | 99.8 | 99.9 |
| **SEQ ID NO: 53** | 95.5 | 99.7 |

**Example 8**

Application of imine reductase mutants labeled as '+++++' in Table **2** in the preparation of S-(3-pyrrolidin-2-yl)pyridine

In a 10 mL reaction vial, 1000 mg of myosmine was added as a substrate, and 4 mL of a phosphate buffer (pH 6.0, 0.1 M) was mixed in. Subsequently, the resulting mixture was adjusted to a pH of 6.0. Then, 5 mg of NADP and 1800 mg of glucose were added and stirred until complete dissolution was achieved. Then, 10 mg of glucose dehydrogenase and 50 mg of the powder of the imine reductase mutant were added, mixed, raised to a temperature of 25°C, and stirred at 300 rpm for 16 hours. After the reaction was completed, 100 μL of the reaction solution was taken and vigorously mixed with 900 μL of acetonitrile. The mixture was then filtered using a 0.22 μm filter membrane for HPLC analysis. 500 μL of the reaction solution was taken and adjusted to a pH exceeding 11, followed by extraction using 1000 μL of n-hexane. The extracted n-hexane layer was filtered through a membrane and used for chiral HPLC analysis.

The conversion rates and enantiomeric excess (ee%) for **SEQ ID NO: 37** and three mutants exhibiting the

highest activity are shown in Table **6.**

Table **6**

| Enzyme | Conversion Rate | ee% |
|---|---|---|
| **SEQ ID NO: 37** | 46.8 | 99.7 |
| **SEQ ID NO: 61** | 99.3 | 99.8 |
| **SEQ ID NO: 65** | 99.5 | 99.7 |
| **SEQ ID NO: 71** | 99.9 | 99.6 |

**Example 9**

Application of imine reductase mutants labeled as '++++++' in Table **2** in the preparation of S-(3-pyrrolidin-2-yl)pyridine

In a 20 mL reaction vial, 3 g of myosmine was added as a substrate, and 7 mL of a phosphate buffer (pH 6.0, 0.1 M) was mixed in. Subsequently, the resulting mixture was adjusted to a pH of 6.0. Then, 20 mg of NADP and 5.4 g of glucose were added and stirred until complete dissolution was achieved. Then, 20 mg of glucose dehydrogenase and 100 mg of the powder of the imine reductase mutant were added, thoroughly mixed, raised to a temperature of 25°C, and stirred at 300 rpm for 16 hours. After the reaction was completed, 100 μL of the reaction solution was taken and vigorously mixed with 900 μL of acetonitrile. The mixture was then filtered using a 0.22 μm filter membrane for HPLC analysis. 500 μL of the reaction solution was taken and adjusted to a pH exceeding 11, followed by extraction using 1000 μL of n-hexane. The extracted n-hexane layer was filtered through a membrane and used for chiral HPLC analysis.

The conversion rates and enantiomeric excess (ee%) for **SEQ ID NO: 37** and four mutants exhibiting the highest activity are shown in Table **7.**

Table **7**

| Enzyme | Conversion Rate | ee% |
|---|---|---|
| **SEQ ID NO: 37** | 33.3 | 99.8 |
| **SEQ ID NO: 81** | 99.1 | 99.9 |
| **SEQ ID NO: 85** | 99.6 | 99.6 |
| **SEQ ID NO: 95** | 99.9 | 99.9 |
| **SEQ ID NO: 133** | 99.9 | 100 |

**Example 10**

Screening of imine reductase mutants in Table **2** using 4-(methylamino)-1-(pyridin-3-yl)butan-1-one as a substrate

In a 5 mL centrifuge tube, 30 mg of 4-(methylamino)-1-(pyridin-3-yl)butan-1-one was added as a substrate, and 2 mL of a phosphate buffer (pH 6.0, 0.1 M) was mixed in. Subsequently, 0.3 mg of NADP and 50 mg of glucose were added and stirred until complete dissolution was achieved. Then, 3 mg of glucose dehydrogenase and 30 mg of the powder of the imine reductase mutant were added, thoroughly mixed, raised to a temperature of 25°C, and stirred at 300 rpm for 24 hours. After the reaction was completed, 100 μL of the reaction solution was taken and vigorously mixed with 900 μL of acetonitrile. The mixture was then filtered using a 0.22 μm filter membrane for HPLC analysis. The conversion rate was calculated by analyzing the area ratio of (S)-nicotine. In cases of high conversion rates, 1 mL of the reaction solution was extracted with ethyl acetate to measure the enantiomeric excess (ee%).

The conversion rates and enantiomeric excess (ee%) for **SEQ ID NO: 1** and eleven mutants with higher activity are presented in Table **8.**

| Enzyme | Conversion Rate | ee% |
|---|---|---|
| **SEQ ID NO: 1** | 50.8 | 98.8 |
| **SEQ ID NO: 23** | 98.1 | 99.3 |

(continued)

| Enzyme | Conversion Rate | ee% |
|---|---|---|
| **SEQ ID NO: 25** | 99.7 | 99.8 |
| **SEQ ID NO: 27** | 99.7 | 99.9 |
| **SEQ ID NO: 37** | 100 | 100 |
| **SEQ ID NO: 43** | 99.7 | 99.4 |
| **SEQ ID NO: 45** | 99.1 | 99.5 |
| **SEQ ID NO: 47** | 100 | 99.8 |
| **SEQ ID NO: 61** | 100 | 100 |
| **SEQ ID NO: 65** | 99.8 | 99.6 |
| **SEQ ID NO: 73** | 99.7 | 99.8 |
| **SEQ ID NO: 85** | 99.9 | 100 |

### Example 11

Application of an imine reductase mutant comprising the amino acid sequence represented by **SEQ ID NO: 37** in the preparation of (S)-nicotine.

4.5 g of 4-(methylamino)-1-(pyridin-3-yl)butan-1-one and 20 mL of 0.1 M phosphate buffer was added to a 50 mL three-neck round-bottom flask, and the pH of the mixture was adjusted to 7.0. Subsequently, 4.8 g of glucose was added to a reaction vial and stirred until complete dissolution was achieved. Then, 0.04 g of glucose dehydrogenase and 0.008 g of NADP salt were added to the reaction vial and stirred until fully dissolved. The solution from the reaction vial was then slowly added to the three-neck round-bottom flask. The reaction was conducted at 30°C with continuous stirring at 300 rpm for 16 hours. The resulting product was collected and has a conversion rate of 99%. The reaction was then stopped and the resulting product was filtered. The filtrate was adjusted to pH=10 using sodium hydroxide solution, extracted with methyl tert-butyl ether, dried with anhydrous sodium sulfate, and concentrated to yield 2.6 g of (S)-nicotine. The obtained (S)-nicotine exhibited a purity of 99% and an optical purity of 100%.

The obtained (S)-nicotine was characterized by proton nuclear magnetic resonance spectroscopy (1H-NMR), and the NMR data results were as follows: 1H-NMR (400 MHz, CDCl3): $\delta$ ppm 8.54(1H, d), 8.50(1H, dd), 7.70 (1H, dt), 7.24-7.27 (1H, m), 3.22-3.27(1H, m), 3.08(1H, t), 2.27-2.34(1H, m), 2.17-2.24(1H, m), 2.16(3H, m), 1.91-2.02(1H, m), 1.79-1.87(1H, m), 1.68-1.76(1H, m). The results confirm the successful synthesis of (S)-nicotine.

### Example 12

Application of an imine reductase mutant comprising the amino acid sequence of **SEQ ID NO: 47** in the preparation of (S)-nicotine.

4.5 g of 4-(methylamino)-1-(pyridin-3-yl)butan-1-one and 20 mL of 0.1 M phosphate buffer was added to a 50 mL three-neck round-bottom flask, and the pH of the mixture was adjusted to 7.0. Subsequently, 4.8 g of glucose was added to the three-neck round-bottom flask and stirred until complete dissolution was achieved. Then, 10 mL of 0.1 M phosphate buffer, 0.3 g of the imine reductase mutant comprising the amino acid sequence represented by **SEQ ID NO: 47,** 0.04 g of glucose dehydrogenase and 0.008 g of NADP salt were added to another 50 mL flask and stirred until fully dissolved. The solution from the flask was then slowly added to the three-neck round-bottom flask. The reaction was conducted at 30°C with continuous stirring at 300 rpm for 16 hours. After the reaction was completed, 100 $\mu$L of the reaction solution was taken and vigorously mixed with 900 $\mu$L of acetonitrile. The mixture was then filtered using a 0.22 $\mu$m filter membrane for HPLC analysis. The conversion rate was calculated by analyzing the area ratio of (S)-nicotine. 1 mL of the reaction solution was extracted with ethyl acetate to measure the enantiomeric excess (ee%). The results showed that the conversion rate was 99.5%, and the ee value was 99.6%.

### Example 13

Application of an imine reductase mutant comprising the amino acid sequence of **SEQ ID NO: 61** in the preparation of (S)-nicotine.

4.5 g of 4-(methylamino)-1-(pyridin-3-yl)butan-1-one and 20 mL of 0.1 M phosphate buffer was added to a 50

mL three-neck round-bottom flask, and the pH of the mixture was adjusted to 7.0. Subsequently, 4.8 g of glucose was added to the three-neck round-bottom flask and stirred until complete dissolution was achieved. Then, 10 mL of 0.1 M phosphate buffer, 0.3 g of the imine reductase mutant comprising the amino acid sequence represented by **SEQ ID NO: 61,** 0.04 g of glucose dehydrogenase and 0.008 g of NADP salt were added to another 50 mL flask and stirred until fully dissolved. The solution from the flask was then slowly added to the three-neck round-bottom flask. The reaction was conducted at 30°C with continuous stirring at 300 rpm for 16 hours. After the reaction was completed, 100 μL of the reaction solution was taken and vigorously mixed with 900 μL of acetonitrile. The mixture was then filtered using a 0.22 μm filter membrane for HPLC analysis. The conversion rate was calculated by analyzing the area ratio of (S)-nicotine. 1 mL of the reaction solution was extracted with ethyl acetate to measure the enantiomeric excess (ee%). The results showed that the conversion rate was 93.2%, and the ee value was 99.7%.

**Example 14**

Application of an imine reductase mutant comprising the amino acid sequence of **SEQ ID NO: 85** in the preparation of (S)-nicotine.

4.5 g of 4-(methylamino)-1-(pyridin-3-yl)butan-1-one and 20 mL of 0.1 M phosphate buffer was added to a 50 mL three-neck round-bottom flask, and the pH of the mixture was adjusted to 7.0. Subsequently, 4.8 g of glucose was added to the three-neck round-bottom flask and stirred until complete dissolution was achieved. Then, 10 mL of 0.1 M phosphate buffer, 0.3 g of the imine reductase mutant comprising the amino acid sequence represented by **SEQ ID NO: 85,** 0.04 g of glucose dehydrogenase and 0.008 g of NADP salt were added to another 50 mL flask and stirred until fully dissolved. The solution from the flask was then slowly added to the three-neck round-bottom flask. The reaction was conducted at 30°C with continuous stirring at 300 rpm for 16 hours. After the reaction was completed, 100 μL of the reaction solution was taken and vigorously mixed with 900 μL of acetonitrile. The mixture was then filtered using a 0.22 μm filter membrane for HPLC analysis. The conversion rate was calculated by analyzing the area ratio of (S)-nicotine. 1 mL of the reaction solution was extracted with ethyl acetate to measure the enantiomeric excess (ee%). The results showed that the conversion rate was 100%, and the ee value was 99.8%.

**Example 15**

Preparation of a powder of a co-expressed recombinant enzyme

A gene, encoding glucose dehydrogenase, was amplified from the pET30a-GDH vector (comprising an amino acid sequence represented by **SEQ ID NO: 171**). The amplification was performed using a pair of primers comprising restriction enzyme sites (as listed in Table **9**). Subsequently, the pET30a vector containing the imine reductase mutant gene fragment and the amplified glucose dehydrogenase gene fragment were digested using two restriction enzymes, BamHI and XhoI. The digested gene fragments were recovered from an agarose gel and ligated using T4 DNA ligase. The ligation product was then transformed into Escherichia coli BL21 (DE3) competent cells, yielding recombinant bacteria comprising both the imine reductase gene and the glucose dehydrogenase gene (a plasmid map can be found in FIG. **9**).

Table **9:** Primer sequences for amplification of glucose dehydrogenase

| Sequence number | Primer sequence |
|---|---|
| **SEQ ID NO: 171** | GCCGGATCCAATAATTTTGTTTAACTTTAAGAAGG |
| **SEQ ID NO: 172** | GGCTCGAGTTAGCCACGACCCGCTTGAAAGC |

The obtained recombinant bacteria were inoculated into 5 mL of LB liquid culture medium containing 50 μg/mL kanamycin and incubated overnight at 37°C. Then, 1 mL of the bacterial culture was transferred into 100 mL of LB liquid culture medium containing 50 μg/mL kanamycin and incubated at 37°C for 3 hours. Subsequently, 50 μL of 1 M IPTG was added, and the culture was further incubated for 16 hours at 25°C. The bacterial cells were collected by centrifugation (at 4000 rpm and 4°C for 10 minutes) and resuspended in PBS buffer (with a pH=7.0) at a volume four times that of the bacterial cell pellet. The suspension was ultrasonically treated, and the resulting solution was freeze-dried to yield a powder of a co-expressed recombinant enzyme that concurrently expresses both the imine reductase and glucose dehydrogenase.

**Example 16**

Application of a powder of a co-expressed recombinant enzyme in the preparation of S-(3-pyrrolidin-2-yl)pyridine

In a 10 mL reaction vial, 750 mg of myosmine was added as a substrate, and 4 mL of phosphate buffer (pH 6.0, 0.1 M) was mixed in. Subsequently, the resulting mixture was adjusted to a pH of 6.0. Then, 5 mg of NADP and 1200 mg of glucose were added and stirred until complete dissolution was achieved. Then, the powder of the co-expressed recombinant enzyme (notably, the imine reductase mutant comprises a nucleic acid sequence represented by **SEQ ID NO: 37,** and the nucleic acid sequence of the co-expressed recombinant enzyme is shown in **SEQ ID NO: 173**) was added, mixed, raised to a temperature of 25°C, and stirred at 300 rpm for 16 hours. After the reaction was completed, 100 μL of the reaction solution was taken and vigorously mixed with 900 μL of acetonitrile. The mixture was then filtered using a 0.22 μm filter membrane for HPLC analysis. 500 μL of the reaction solution was taken and adjusted to a pH exceeding 11, followed by extraction using 1000 μL of n-hexane. The extracted n-hexane layer was filtered through a membrane and used for chiral HPLC analysis. The results showed that the conversion rate was 99.6%, and the ee value was 99.8%.

**Example 17**

Application of a powder of a co-expressed recombinant enzyme in the preparation of (S)-nicotine.

4.5 g of 4-(methylamino)-1-(pyridin-3-yl)butan-1-one and 30 mL of 0.1 M phosphate buffer was added to a 50 mL three-neck round-bottom flask, and the pH of the mixture was adjusted to 7.0. Subsequently, 4.8 g of glucose and 0.008 g of NADP salt were added to the three-neck round-bottom flask and stirred until complete dissolution was achieved. Then, 0.3 g of the powder of co-expressed recombinant enzyme (notably, the imine reductase mutant comprises a nucleic acid sequence represented by **SEQ ID NO: 37,** and the nucleic acid sequence of the co-expressed recombinant enzyme is shown in **SEQ ID NO: 173**) was added, stirred until fully dissolved. The reaction was conducted at 30°C with continuous stirring at 300 rpm for 16 hours. After the reaction was completed, 100 μL of the reaction solution was taken and vigorously mixed with 900 μL of acetonitrile. The mixture was then filtered using a 0.22 μm filter membrane for HPLC analysis. The conversion rate was calculated by analyzing the area ratio of (S)-nicotine. 1 mL of the reaction solution was extracted with ethyl acetate to measure the enantiomeric excess (ee%). The results showed that the conversion rate was 99.6%, and the ee value was 99.8%.

**Example 18**

Preparation of (S)-nicotine from (S)-nornicotine

(S)-nicotine was prepared from (S)-nornicotine obtained in Examples **5-9** or **16.**

107 g of (S)-nornicotine from Example **16** and 80 g of 37% formaldehyde solution were added to three 500 mL three-necked flasks. The temperature was increased to 75°C. Then, 60 g of 85% formic acid solution was added dropwise, and the reaction was maintained at 75°C for 24 hours. After the reaction was completed, sodium hydroxide was added to adjust the pH of the reaction solution to 12. The aqueous phase was extracted with methyl tert-butyl ether, and the combined extract was concentrated and subjected to vacuum distillation to yield 80 g of colorless liquid, which is (S)-nicotine.

The obtained product was characterized by proton nuclear magnetic resonance spectroscopy (1H-NMR), and the NMR data results were as follows: 1H-NMR (400 MHz, CDCl3): δ ppm 8.54(1H, d), 8.50(1H, dd), 7.70 (1H, dt), 7.24-7.27 (1H, m), 3.22-3.27(1H, m), 3.08(1H, t), 2.27-2.34(1H, m), 2.17-2.24(1H, m), 2.16(3H, m), 1.91-2.02(1H, m), 1.79-1.87(1H, m), 1.68-1.76(1H, m). This confirms the successful synthesis of (S)-nicotine.

It will be obvious to those skilled in the art that changes and modifications may be made, and therefore, the aim in the appended claims is to cover all such changes and modifications.

## Claims

1. An imine reductase mutant, comprising a mutation in an amino acid sequence represented by **SEQ ID NO: 1,** wherein,
   the mutation in the amino acid sequence comprises V171, A172, Y230, or a combination thereof.

2. The imine reductase mutant of claim 1, wherein the mutation in the amino acid sequence comprises V171Y/N/A/S, A172V/F, Y230G/A/T, or a combination thereof.

**3.** The imine reductase mutant of claim 1 or 2, wherein the imine reductase mutant comprises one of amino acid sequences selecting from a group consisting of **SEQ ID NO: 3, SEQ ID NO: 5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 55, SEQ ID NO: 57, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 87, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 105, SEQ ID NO: 107, SEQ ID NO: 109, SEQ ID NO: 111, SEQ ID NO: 113, SEQ ID NO: 115, SEQ ID NO: 117, SEQ ID NO: 119, SEQ ID NO: 121, SEQ ID NO: 123, SEQ ID NO: 125, SEQ ID NO: 127, SEQ ID NO: 129, SEQ ID NO: 131, SEQ ID NO: 133, SEQ ID NO: 135, SEQ ID NO: 137, SEQ ID NO: 139, SEQ ID NO: 141, SEQ ID NO: 143, SEQ ID NO: 145, SEQ ID NO: 147, SEQ ID NO: 149, SEQ ID NO: 151, SEQ ID NO: 153, SEQ ID NO: 155, SEQ ID NO: 157, SEQ ID NO: 159, SEQ ID NO: 161, SEQ ID NO: 163, SEQ ID NO: 165,** and **SEQ ID NO: 167.**

**4.** A method for preparing (S)-nicotine, and the method comprising: under suitable conditions, catalytically reducing a substrate I to (S)-nornicotine by the imine reductase mutant of any one of claims 1, 2, and 3, and methylating (S)-nornicotine to yield (S)-nicotine;
the substrate I being shown in formula I and (S)-nornicotine being shown in formula III:

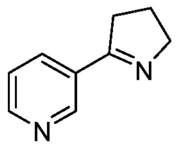

I ;

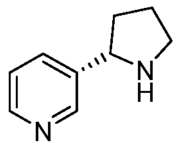

III .

**5.** The method of claim 4, wherein the substrate I, a coenzyme, glucose, glucose dehydrogenase, a buffer solution, and the imine reductase mutant are mixed to form (S)-nornicotine; and (S)-nornicotine is methylated to yield (S)-nicotine.

**6.** A method for preparing (S)-nicotine, and the method comprising: under suitable conditions, catalytically reducing a substrate II to yield (S)-nicotine by the imine reductase mutant of any one of claims 1, 2, and 3;
the substrate II being shown in formula II:

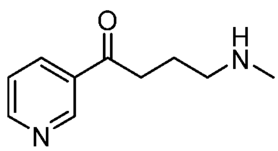

II .

**7.** The method of claim 6, wherein the substrate II is cyclized and then catalytically reduced by the imine reductase mutant to yield (S)-nicotine, or;

a slat of the substrate II is desalted, cyclized, and catalytically reduced by the imine reductase mutant to yield (S)-nicotine; and
the salt of the substrate II comprises hydrochloride, dihydrochloride, hydrobromide, dihydrobromide, sulphate or hydrogen sulphate.

**8.** A nucleic acid sequence, encoding the imine reductase mutant of any one of claims 1, 2, and 3.

**9.** An expression vector, comprising the nucleic acid sequence of claim 8.

**10.** A vector cell, comprising the nucleic acid sequence of claim 8 or the expression vector of claim 9.

**11.** A method for preparing the imine reductase mutant of any one of claims 1, 2, and 3, and the method comprising cultivating a cell to generate the imine reductase mutant.

**12.** The method of claim 11, wherein the imine reductase mutant and glucose dehydrogenase are simultaneously expressed through a co-expressed recombinant enzyme.

**13.** The method of claim 12, wherein the co-expressed recombinant enzyme is generated by a cell comprising both an imine reductase mutant gene fragment and a glucose dehydrogenase gene fragment.

**14.** The method of claim 13, comprising: amplifying the glucose dehydrogenase gene fragment from a vector using a pair of primers comprising specific restriction enzyme recognition sites; respectively digesting the imine reductase mutant gene fragment on an expression vector and the amplified glucose dehydrogenase gene fragment with two restriction enzymes, BamHI and XhoI; recovering the two digested gene fragments from an agarose gel; ligating the two digested gene fragments using T4 DNA ligase to form a ligated DNA; transforming the ligated DNA into Escherichia coli BL21 competent cells, thus obtaining recombinant bacteria containing both the imine reductase mutant gene fragment and the glucose dehydrogenase gene fragment; cultivating, inducing, and centrifuging the recombinant bacteria and collecting bacterial cells; resuspending and ultrasonically breaking the bacterial cells; and freeze-drying a resulting solution to obtain powders of the co-expressed recombinant enzyme.

**15.** An imine reductase mutant prepared by the method of any one of claims 11-14 or a co-expressed recombinant enzyme comprising the same.

**16.** A method for preparing (S)-nicotine by the co-expressed recombinant enzyme of claim 15, the method comprising, under suitable conditions, catalytically reducing a substrate I to (S)-nornicotine by the co-expressed recombinant enzyme, and methylating (S)-nornicotine to yield (S)-nicotine;
the substrate I being shown in formula I and (S)-nornicotine being shown in formula III:

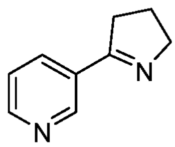
I ;
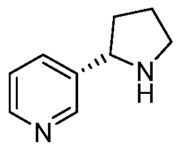
III .

**17.** A method for preparing (S)-nicotine by the co-expressed recombinant enzyme of claim 15, the method comprising, under suitable conditions, catalytically reducing a substrate II to yield (S)-nicotine;
the substrate II being shown in formula II:

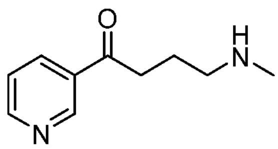
II .

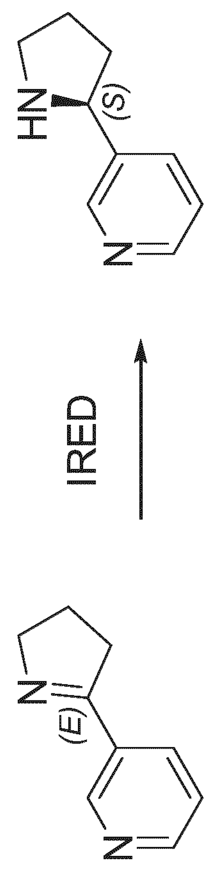


3-(3,4-dihydro-2*H*-pyrrol-5-yl)pyridine

(*S*)-3-(pyrrolidin-2-yl)pyridine

FIG. 1

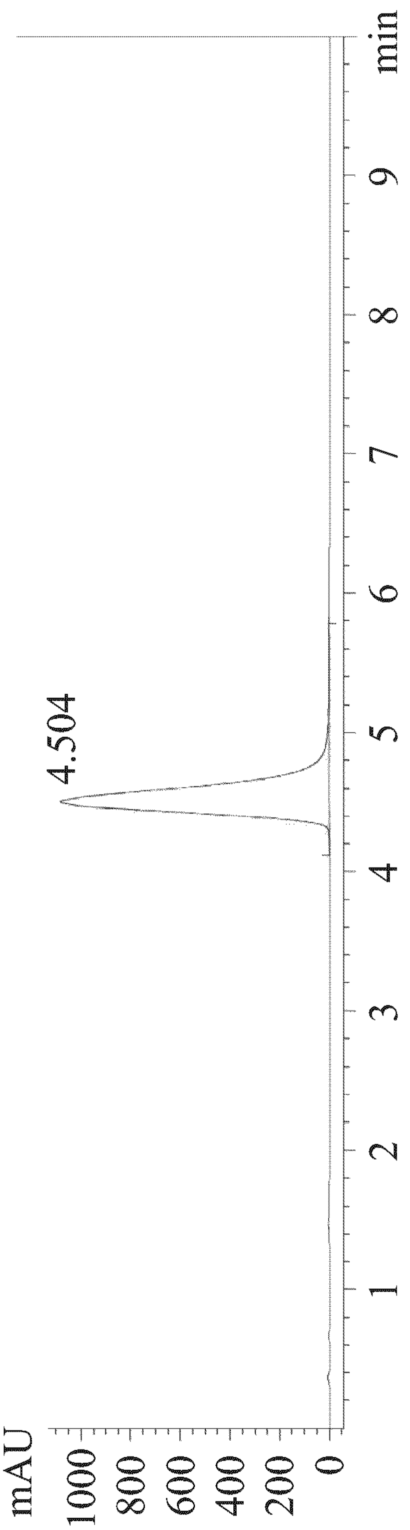


FIG. 2

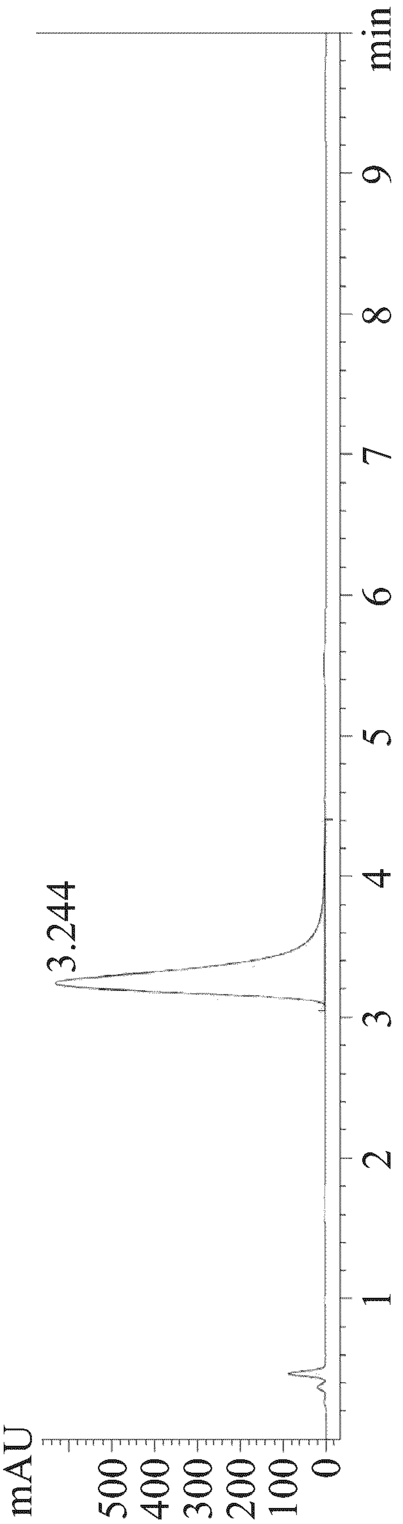


FIG. 3

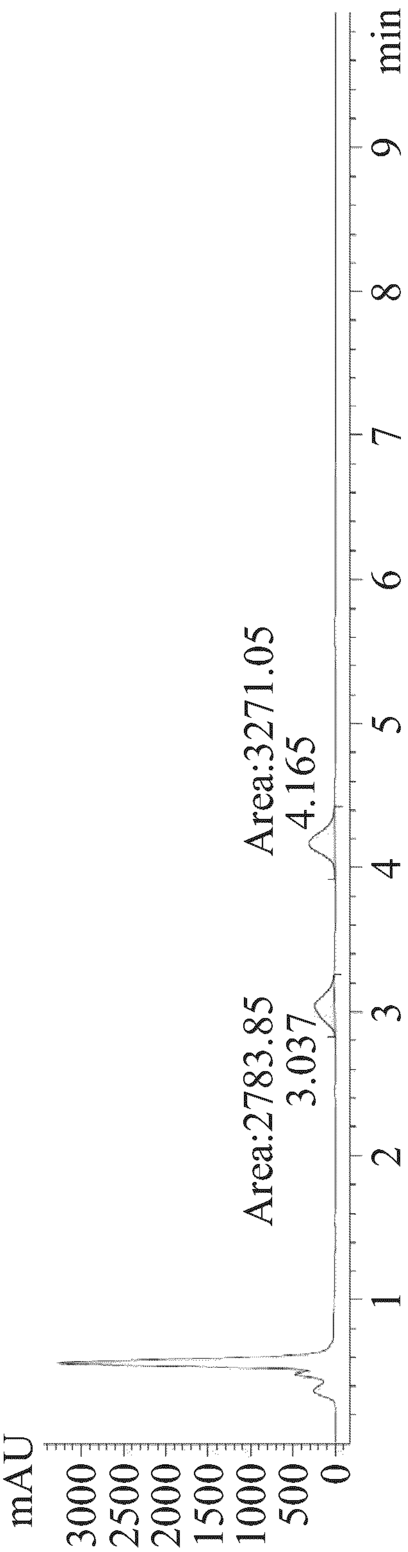
mAU
3000
2500
2000
1500
1000
500
0
1
2
3
4
5
6
7
8
9
min
Area:2783.85
3.037
Area:3271.05
4.165

FIG. 4

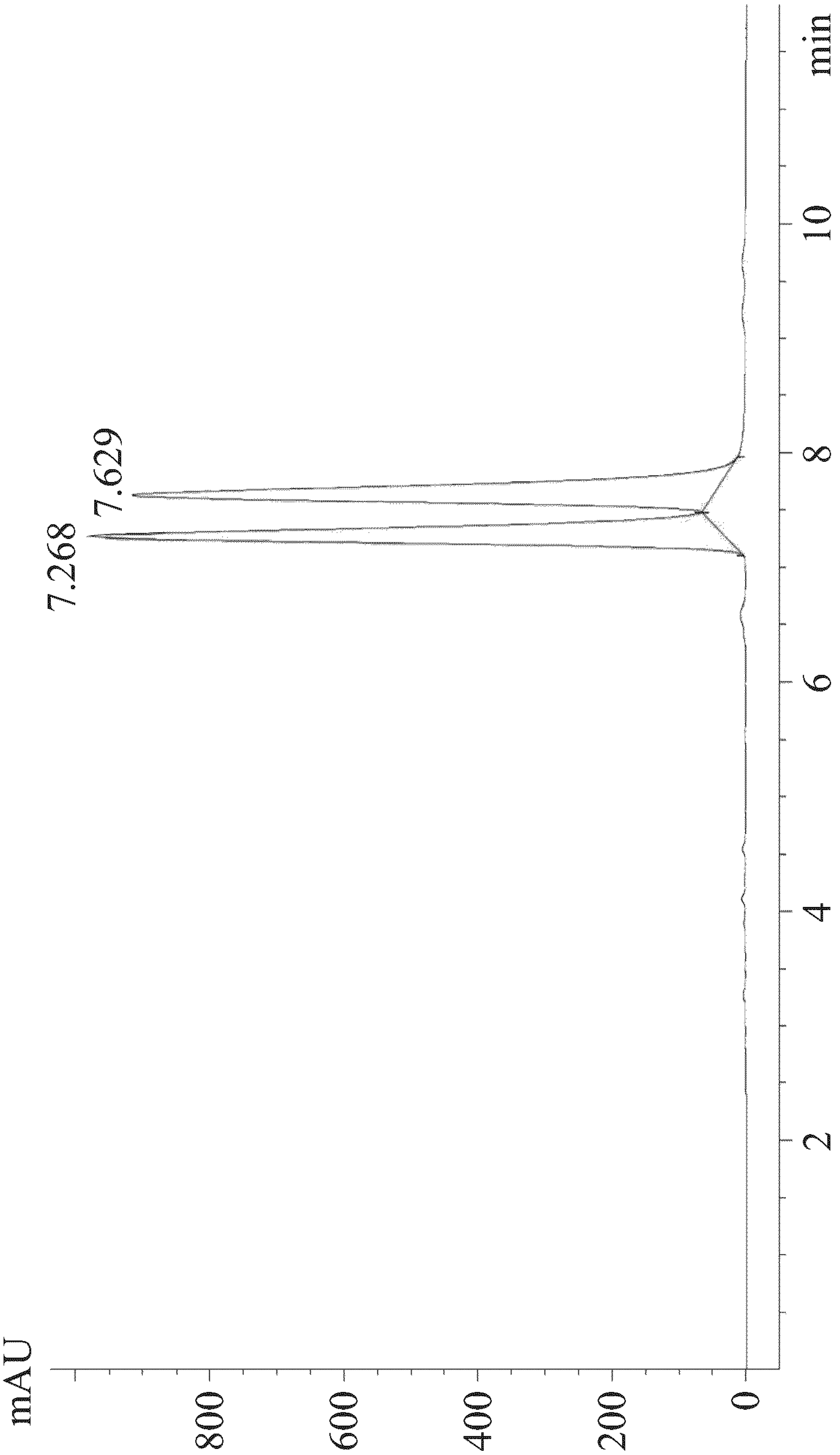


FIG. 5

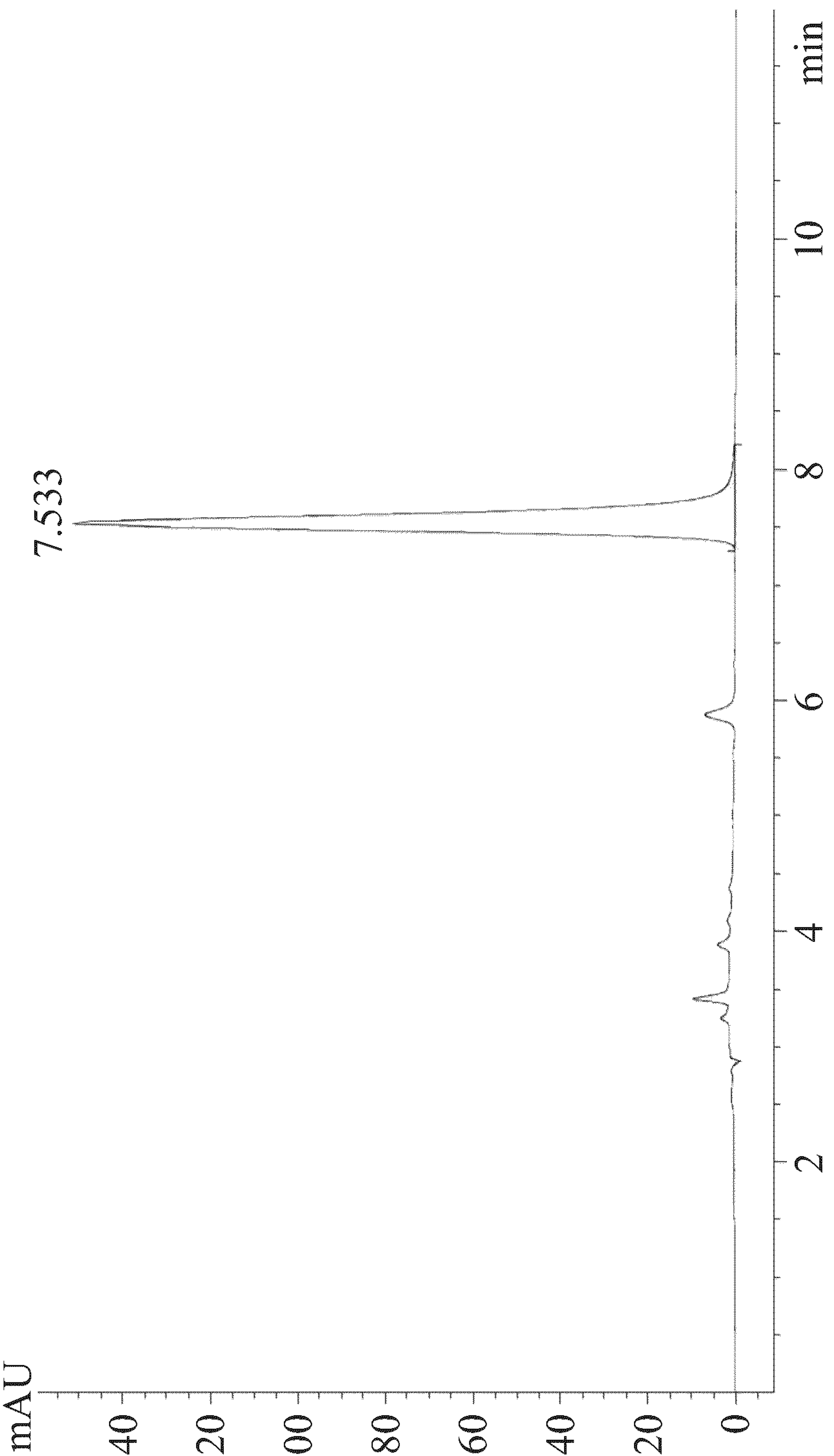


FIG. 6

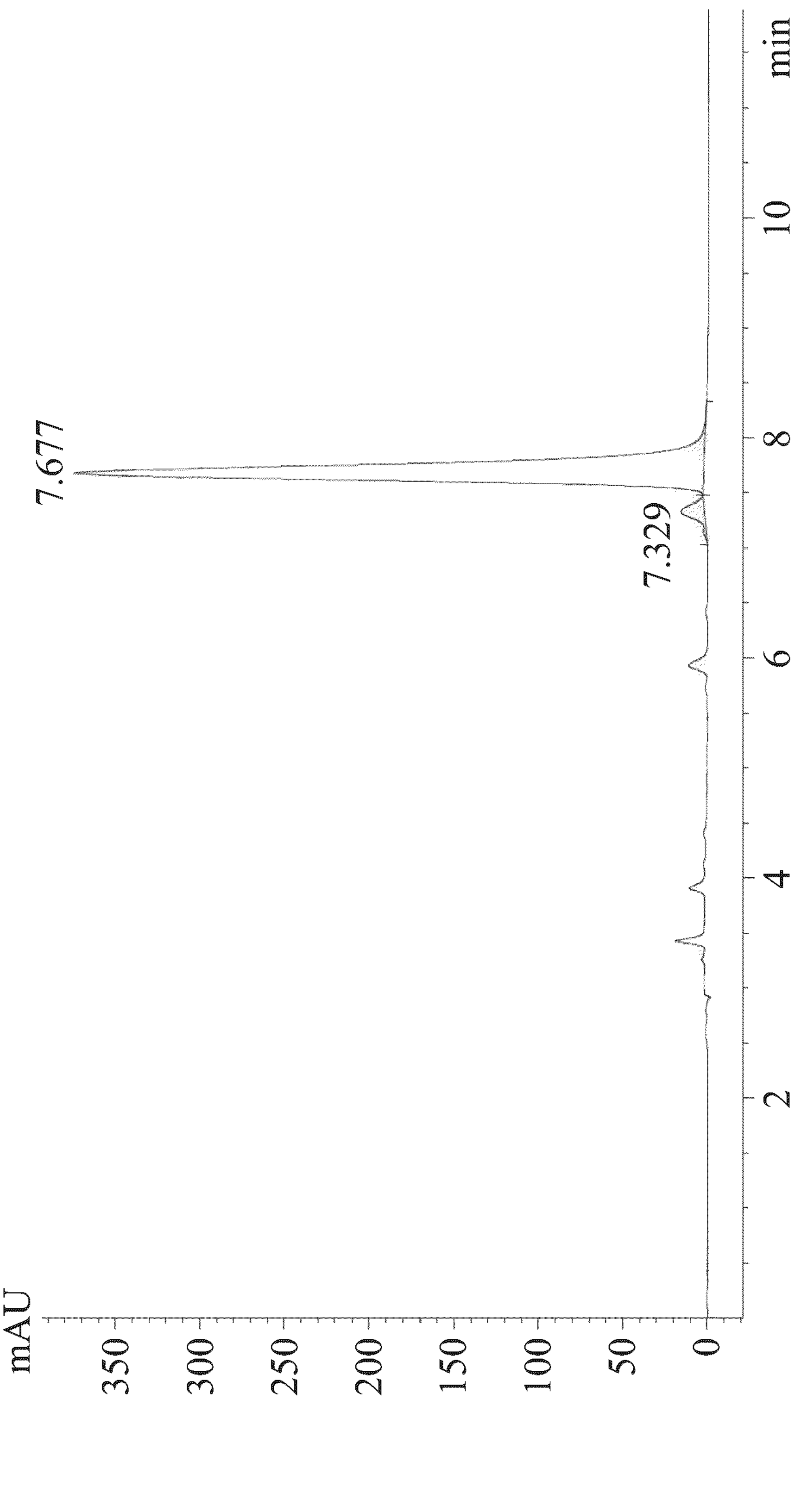


FIG. 7

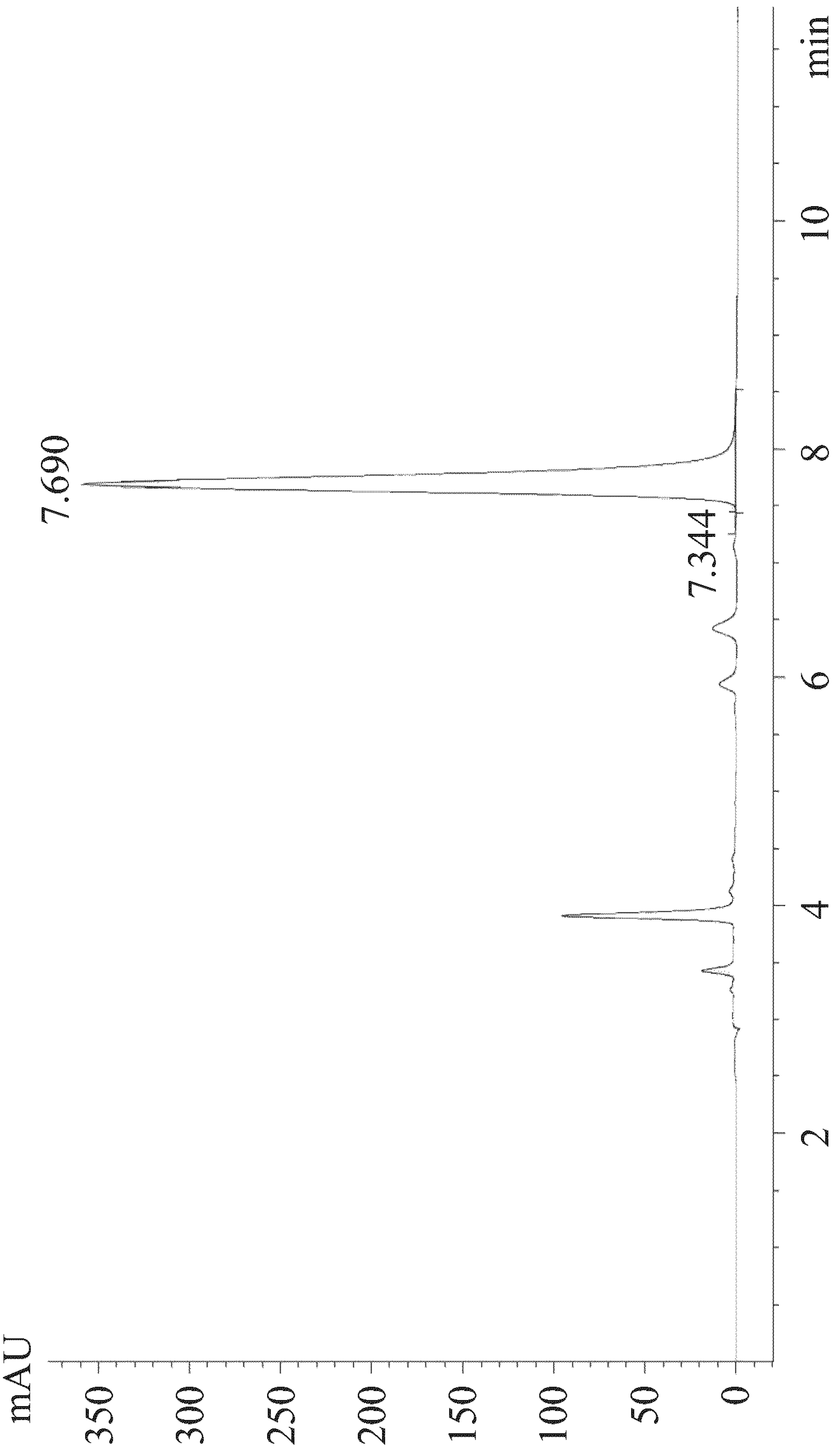


FIG. 8

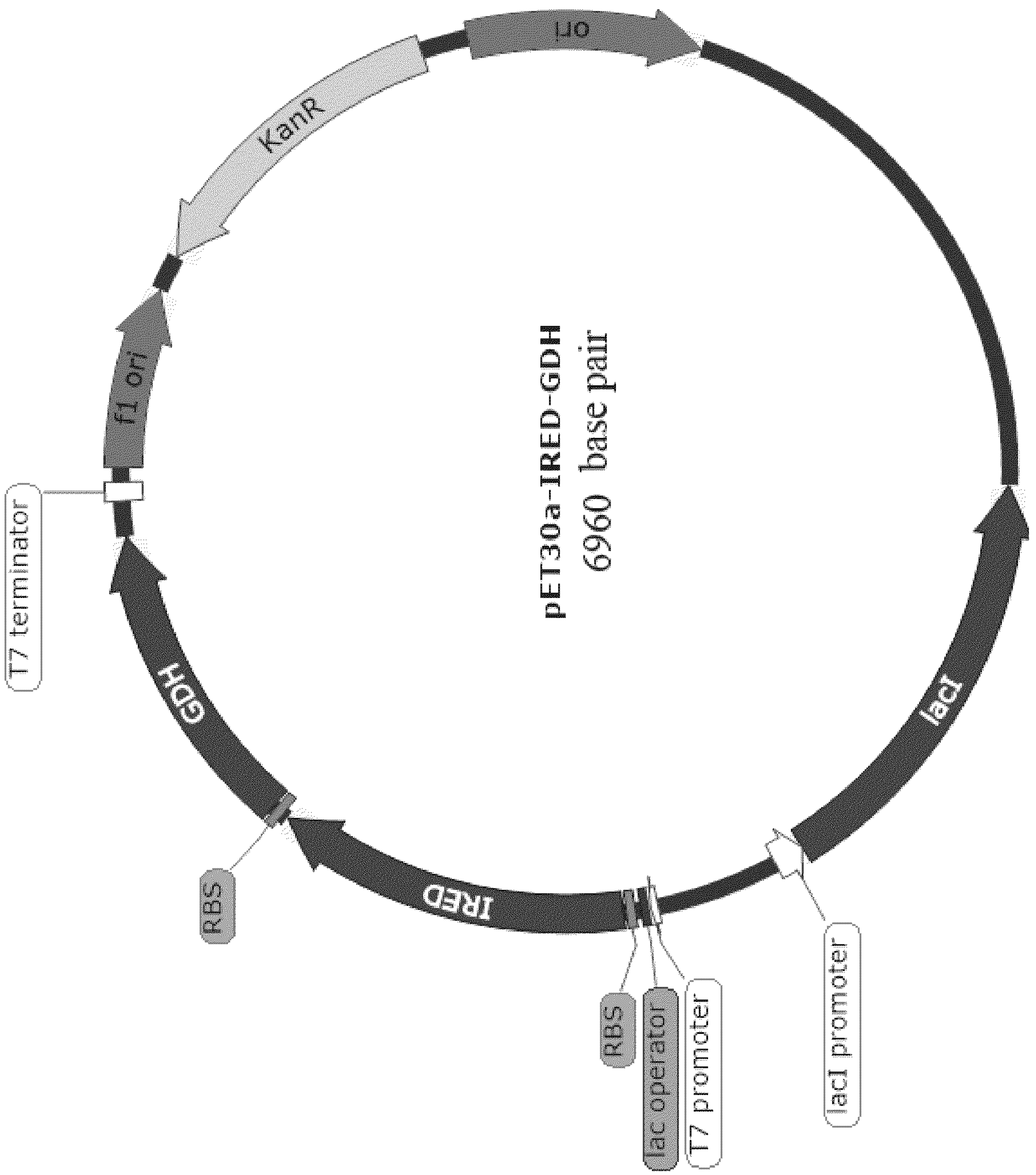
pET30a-IRED-GDH
6960 base pair
ori
KanR
f1 ori
T7 terminator
GDH
RBS
IRED
RBS
lac operator
T7 promoter
lacI promoter
lacI

FIG. 9

**INTERNATIONAL SEARCH REPORT**

International application No.
**PCT/CN2022/099711**

**A. CLASSIFICATION OF SUBJECT MATTER**

C12N 9/06(2006.01)i；C12N 15/53(2006.01)i；C12N 11/089(2020.01)i；C12N 1/21(2006.01)i；C12P 17/16(2006.01)i

According to International Patent Classification (IPC) or to both national classification and IPC

**B. FIELDS SEARCHED**

Minimum documentation searched (classification system followed by classification symbols)

C12N; C12P

Documentation searched other than minimum documentation to the extent that such documents are included in the fields searched

Electronic data base consulted during the international search (name of data base and, where practicable, search terms used)

DWPI, CNTXT, WPABSC, ENTXT, OETXT, CNKI, PubMed, Elsevier Science , ISI WEB of Science; GenBank+EMBL+DDBJ ; 中国专利生物序列检索系统, Chinese Patent Biological Sequence Retrieval System: imine reductase, 亚胺还原酶, myosmine, 肌胺, 麦斯明, 麦思明, (S)-nornicotine, (S)-降烟碱, (S)-去甲烟碱, (S)-尼古丁, (S)-nicotine, V171, V171Y, V171N, V171A, V171S, 对SEQ ID NO: 1的检索, search for SEQ ID NO: 1, 对发明人的追踪检索, tracking search for the inventor

**C. DOCUMENTS CONSIDERED TO BE RELEVANT**

| Category* | Citation of document, with indication, where appropriate, of the relevant passages | Relevant to claim No. |
|---|---|---|
| X | "NAD(P)-binding Domain-containing Protein [Aeromonas veronii]" *NCBI Reference Sequence: WP_005363076.1,* 01 June 2021 (2021-06-01), see amino acid sequence and related information | 1 (in part), 8-11 (all in part), 15 (in part) |
| A | "NAD(P)-binding Domain-containing Protein [Aeromonas veronii]" *NCBI Reference Sequence: WP_005335883.1,* 01 June 2021 (2021-06-01), see amino acid sequence and related information | 1-17 (all in part) |
| A | 李骥璇 (LI, Jixuan). "亚胺还原酶辅酶再生体系的构建及定点突变的研究 (Co-expressed System Construction and Site-directed Mutagenesis of Imine Reductase)" *中国优秀硕士学位论文全文数据库基础科学辑 (Chinese Master's Dissertations Full-text Database, Basic Sciences),* No. 2, 15 February 2021 (2021-02-15), see abstract | 1-17 (all in part) |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 September 2022** | **28 September 2022** |
| Name and mailing address of the ISA/CN<br>**China National Intellectual Property Administration (ISA/CN)**<br>**No. 6, Xitucheng Road, Jimenqiao, Haidian District, Beijing 100088, China**<br>Facsimile No. **(86-10)62019451** | Authorized officer<br><br>Telephone No. |

Form PCT/ISA/210 (second sheet) (January 2015)

**INTERNATIONAL SEARCH REPORT**

| International application No. |
|---|
| **PCT/CN2022/099711** |

**C. DOCUMENTS CONSIDERED TO BE RELEVANT**

| Category* | Citation of document, with indication, where appropriate, of the relevant passages | Relevant to claim No. |
|---|---|---|
| A | 程峰等 (CHENG, Feng et al.). "NAD(P)H依赖型氧化还原酶不对称还原胺化制备手性胺的研究 (NAD(P)H-dependent Oxidoreductases for Synthesis of Chiral Amines by Asymmetric Reductive Amination of Ketones)" *生物工程学报 (Chinese Journal of Biotechnology),* Vol. 36, No. 9, 25 September 2020 (2020-09-25), see abstract | 1-17 (all in part) |
| A | MAN, H. et al. "Structure, Activity and Stereoselectivity of NADPH-Dependent Oxidoreductases Catalysing the S-Selective Reduction of the Imine Substrate 2-Methylpyrroline" *Chembiochem,* Vol. 16, No. 7, 24 March 2021 (2021-03-24), see abstract | 1-17 (all in part) |
| A | CN 110564788 A (JIANGNAN UNIVERSITY) 13 December 2019 (2019-12-13) see abstract | 1-17 (all in part) |
| A | WO 2020098978 A1 (ZANOPRIMA LIFESCIENCES LTD.) 22 May 2020 (2020-05-22) see claims 1-15 | 1-17 (all in part) |

Form PCT/ISA/210 (second sheet) (January 2015)

**INTERNATIONAL SEARCH REPORT**

International application No.
**PCT/CN2022/099711**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   - a. ☑ forming part of the international application as filed:
     - ☑ in the form of an Annex C/ST.25 text file.
     - ☐ on paper or in the form of an image file.
   - b. ☐ furnished together with the international application under PCT Rule 13*ter*.1(a) for the purposes of international search only in the form of an Annex C/ST.25 text file.
   - c. ☐ furnished subsequent to the international filing date for the purposes of international search only:
     - ☐ in the form of an Annex C/ST.25 text file (Rule 13*ter*.1(a)).
     - ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

**INTERNATIONAL SEARCH REPORT**

International application No.
**PCT/CN2022/099711**

**Box No. III Observations where unity of invention is lacking (Continuation of item 3 of first sheet)**

This International Searching Authority found multiple inventions in this international application, as follows:

[1] A first group of inventions: claims 1-17 (all in part), relating to an imine reductase mutant comprising a mutation at an amino acid site V171 corresponding to an amino acid sequence shown in SEQ ID NO: 1, a nucleic acid encoding same, an expression vector, a cell, a co-expressed enzyme, an application, and a preparation method.

[2] A second group of inventions: claims 1-17 (all in part), relating to an imine reductase mutant comprising a mutation at an amino acid site A172 corresponding to an amino acid sequence shown in SEQ ID NO: 1, a nucleic acid encoding same, an expression vector, a cell, a co-expressed enzyme, an application, and a preparation method.

[3] A third group of inventions: claims 1-17 (all in part), relating to an imine reductase mutant comprising a mutation at an amino acid site Y230 corresponding to an amino acid sequence shown in SEQ ID NO: 1, a nucleic acid encoding same, an expression vector, a cell, a co-expressed enzyme, an application, and a preparation method.

[4] Because the prior art document CN107384885A (24 November 2017) has disclosed that an imine reductase can be mutated, and the imine reductase shown in SEQ ID NO: 1 of the present application has been disclosed in Genbank accession number WP_005335883.1. Therefore, inventions 1-3 comprising different mutation sites do not share a same or corresponding special technical feature, and thus do not comply with the requirement of unity as defined in PCT Rule 13.1.

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **1-17 (all in part)**

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

**INTERNATIONAL SEARCH REPORT**
**Information on patent family members**

International application No.
**PCT/CN2022/099711**

| Patent document cited in search report | | | Publication date (day/month/year) | Patent family member(s) | | | Publication date (day/month/year) |
|---|---|---|---|---|---|---|---|
| CN | 110564788 | A | 13 December 2019 | None | | | |
| WO | 2020098978 | A1 | 22 May 2020 | ES | 2836481 | T3 | 25 June 2021 |
| | | | | KR | 20210082532 | A | 05 July 2021 |
| | | | | AU | 2019382117 | B2 | 27 May 2021 |
| | | | | CA | 3118774 | A1 | 22 May 2020 |
| | | | | HU | E051799 | T2 | 29 March 2021 |
| | | | | HR | P20201895 | T1 | 16 April 2021 |
| | | | | JP | 2022507375 | A | 18 January 2022 |
| | | | | DK | 3653617 | T3 | 07 December 2020 |
| | | | | PL | 3653617 | T3 | 06 April 2021 |
| | | | | PT | 3653617 | T | 11 December 2020 |
| | | | | BR | 112021009328 | A2 | 17 August 2021 |
| | | | | CN | 113272289 | A | 17 August 2021 |
| | | | | EP | 3653617 | A1 | 20 May 2020 |
| | | | | SI | 3653617 | T1 | 29 January 2021 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2014174505 A
- WO 2020098978 A


### Non-patent literature cited in the description


- **KOICHI MITSUKURA et al.** *Org. Biomol. Chem.,* 2010, vol. 8, 4533-4535
- **LI JIXUAN et al.** *Biotechnology Bulletin,* 2019, vol. 35 (1), 105-111